# EUROPÄISCHE PATENTSCHRIFT

(11) **EP 1 151 681 B1**
(45) Veröffentlichungstag und Bekanntmachung des Hinweises auf die Patenterteilung: **18.07.2007**
(21) Anmeldenummer: 01110409.8
(22) Anmeldetag: 27.04.2001
(51) Int. Cl.: A24C 5/00

(54) **Vorrichtung zum Herstellen von Produkten und Verfahren zum Steuern einer derartigen Vorrichtung**
Apparatus for manufacturing products and method for controlling such an apparatus
Appareil de fabrication de produits et procédé de commande d'un tel appareil

(30) Priorität: 05.05.2000 DE 10021838
(43) Veröffentlichungstag der Anmeldung: 07.11.2001
(73) Patentinhaber: Focke & Co. (GmbH & Co. KG), 27283 Verden (DE)
(72) Erfinder: Focke, Heinz, 27283 Verden (DE); Schröter, Stephan, 27333 Bücken (DE); Murnikow, Denis, 30163 Hannover (DE)
(74) Vertreter: Bolte, Erich

(56) Entgegenhaltungen:
- EP-A- 0 620 512
- DE-A1- 19 639 352
- DE-A1- 19 740 775
- GB-A- 2 330 667
- US-A- 4 486 098
- US-A- 4 682 038
- US-A- 4 704 603
- US-A- 4 907 608
- US-A- 5 177 930
- US-A- 5 325 305
- US-A- 5 412 926
- US-A- 5 838 572
- US-A- 5 877 959
- US-A- 5 908 459
- US-A- 5 966 897
- G.A. MITCHELL: "PCs are gaining control" CONTROL ENGINEERING INTERNATIONAL, März 1999 (1999-03), Seiten 48-52,
- N. SCHMIDT: "Mit dem Bus vom Sensor zur Steuerung" AUTOMATISIERUNGSTECHNIK, Nr. 7-8, 1999,
- B. PELZER: "Soft-SPS macht Druck" IEE, 1998, Seiten 18-21,
- ULRICH: "Antriebsregler als dezentrale SPS" ETZ, Nr. 15, 1999, Seiten 6-8,
- P. FABBRI, E. SALSI: "Il PLC del futuro" MECCANICA & AUTOMAZIONE, Nr. 25, März 1997 (1997-03), Seiten 130-139,
- V.J. VANDOREN: "PC-based control software runs on schedule" CONTROL ENGINEERING, 1. Januar 1998 (1998-01-01),
- G.A. MITCHELL: "Machine control strategies it's only logical" CONTROL ENGINEERING, 10. Januar 1998 (1998-01-10),
- G.A. MITCHELL: "PCSs adapt, but will they withstand assault of PCs" CONTROL ENGINEERING, 5. Januar 1998 (1998-01-05),

## Beschreibung

Die Erfindung betrifft ein Verfahren zum Steuern einer Vorrichtung zum Herstellen von Produkten, insbesondere Zigaretten, Zigarettenpackungen, Pflastern bzw. Pflasterpackungen, mit einer speicherprogrammierbaren Steuerung - SPS -, einer Achssteuerung zum Steuern von Achsen, insbesondere von Antrieben, Servomotoren, Förder- und/oder Schneidorganen, und einer Visualisierung zum Darstellen von Prozessen und/oder Prozessparametern. Die Erfindung betrifft auch eine derartige Vorrichtung.

Bekannt sind Herstellungsmaschinen, beispielsweise Maschinen zum Herstellen von Zigaretten oder Zigarettenpackungen mit einer Mehrzahl von einzeln steuerbaren Antrieben bzw. Servomotoren. Derartige Maschinen weisen regelmäßig eine eigene speicherprogrammierbare Steuerungseinheit auf, mittels derer diese Antriebe bzw. deren Achsen angesteuert werden. Ferner weisen derartige Maschinen Steuerungseinheiten zur Achssteuerung und eine Einheit zur Visualisierung von Prozessen bzw. Prozessparametern auf, wobei diese Visualisierungseinheit als Schnittstelle zwischen Maschine und Bediener dient, d.h. dass die Visualisierungseinheit auch die Bedienung der Maschine unterstützt.

Bei diesen herkömmlichen Maschinen sind die drei genannten Einheiten, nämlich die speicherprogrammierbare Steuerungseinheit, die Achssteuerungseinheit und die Visualisierungseinheit in physikalisch selbstständigen Geräten realisiert. Die Einheiten stammen regelmäßig von verschiedenen Lieferanten und sind daher nicht unmittelbar aufeinander abgestimmt. Die Einheiten werden zwar miteinander verknüpft. Diese Verknüpfung erfordert jedoch zusätzlichen Arbeitsaufwand bei der Herstellung derartiger Maschinen aber auch im Betrieb der Maschinen. Der Datenaustausch ist nur durch eine Datenanpassung der verschiedenen Einheiten untereinander möglich und erfordert daher einen gewissen "Overhead", d.h. Zusatzaufwand, um die Kommunikation dieser drei Komponenten zu gewährleisten. Hierdurch wird die Entwicklung und Wartung derartiger Maschinen erschwert und die Datenverarbeitung verlangsamt.

Die US 5,966,897 zeigt als Beispiel einer herkömmlichen Steuerung einer Maschine mit den vorgenannten Nachteilen die Steuerung einer Verpackungsmaschine. Es ist dabei ein Steuerungssystem offenbart, das in getrennten Komponenten eine SPS, einen Industrie-PC sowie eine Achssteuerung aufweist.

Der Erfindung liegt die Aufgabe zugrunde, derartige Herstellungsmaschirien bzw. deren Steuerung zu verbessern.

Gelöst wird diese Aufgabe durch ein Verfahren mit den Merkmalen des Anspruches 1 sowie durch eine Vorrichtung mit den Merkmalen des Anspruches 6.

Danach ist ein Verfahren der eingangs genannten Art dadurch gekennzeichnet, dass die SPS, die Achssteuerung und die Visualisierung auf einem gemeinsamen physikalischen Gerät, insbesondere einer gemeinsamen Hardwareplattform, ablaufen. Dabei steuert eine erste SPS-Task zeitkritische Prozesse, eine zweite SPS-Task nicht-kritische Prozesse und eine NC-Task die Achsen, wobei diese Tasks auf eine gemeinsame Datenbasis zugreifen. Eine erfindungsgemäße Vorrichtung der eingangs genannten Art ist dadurch gekennzeichnet, dass die SPS, die Achssteuerung und die Visualisierung auf einem gemeinsamen physikalischen Gerät, insbesondere einer gemeinsamen Hardwareplattform, realisiert sind und dass das physikalische Gerät einen Datenspeicher als gemeinsame Datenbasis für eine erste SPS-Task zur Steuerung zeitkritischer Prozesse, eine zweite SPS-Task zur Steuerung nicht-zeitkritischer Prozesse und eine NC-Task zur Achssteuerung aufweist.

Durch die Realisierung der drei Komponenten SPS, Achssteuerung und Visualisierung auf einem gemeinsamen physikalischen Gerät kann der Kommunikationsaufwand zwischen diesen Komponenten erheblich vereinfacht und reduziert werden. Ein Anpassen der Daten der verschiedenen Einheiten ist nicht mehr notwendig. Hierdurch können Kommunikationsprotokolle zwischen den verschiedenen Komponenten entfallen, was zu einem unmittelbaren Zeitgewinn und zu schnelleren Datenzugriffen führt. Ferner ergibt sich wegen einer sich einstellenden übersichtlicheren Datenstruktur eine Reduzierung möglicher Fehlerquellen bei der Entwicklung, Montage und Wartung derartiger Maschinen.

Ferner erhält man eine offene Struktur des Steuerungssystems, was eine weitgehende Unabhängigkeit der eingesetzten Hardware erlaubt. Hierdurch können die Prozesse bei der Herstellung von Produkten schnell angepasst werden. Durch den Austausch des physikalischen Geräts, beispielsweise aufgrund einer neuentwickelten Generation, ist eine Steigerung der Systemleistung insgesamt möglich und nicht bloß eine Leistungssteigerung einzelner Komponenten, die nicht zu einer Gesamtsystemleistungssteigerung führt.

Im vorliegenden Zusammenhang ist der Begriff "Produkt" im weiteren Sinne zu verstehen. Er schließt sowohl das fertige als auch das unfertige Produkt ein, insbesondere auch Vorprodukte. Die Produkte in diesem Sinne durchlaufen verschiedene Prozesse, insbesondere entlang eines oder mehrerer Förderwege. Sowohl die Fördergeschwindigkeit als auch die Verarbeitungsgeschwindigkeit innerhalb der einzelnen Prozesse ist bei derartigen Maschinen regelmäßig sehr hoch. Eine exakte Abstimmung der Prozesse ist daher notwendig, wenn eine hohe Produktqualität erzielt werden soll. Die Erfindung ermöglicht diese exakte und permanente Abstimmung auch bei hohen Geschwindigkeiten.

Die durch die erfindungsgemäße Integration von SPS, Achssteuerung und Visualisierung in einem gemeinsamen physikalischen Gerät erzielte Steigerung der Datenverarbeitungsgeschwindigkeit ermöglicht nämlich die einzelnen Servoantriebe permanent geregelt aufeinander abzustimmen. Hierzu kommen Sensoren zum Einsatz, die permanent die Produkte kontrollieren, wobei die Antriebsachsen in Abhängigkeit von Signalen dieser Sensoren geregelt werden. Eine manuelle Kontrolle, wie bei bekannten Maschinen, kann somit entfallen. Ebenso kann ein manuelles Nachstellen der Servoantriebe entfallen, da dies nunmehr automatisch im Sinne einer Regelung erfolgt.

Eine selbstständige Besonderheit besteht darin, einen Behandlungsprozess, insbesondere ein Besprühen oder Beleimen, einer Materialbahn oder eines Produkts zu unterbrechen, falls ein Sensor eine planmäßige und/oder unplanmäßige Fehlstelle der Materialbahn bzw. des Produkts anzeigt. Eine derartige Unterbrechung erfolgt gezielt nur während des Vorbeiförderns der Fehlstelle an einem Behandlungsorgan, insbesondere seiner Düse, und nicht für die Dauer des Vorbeiförderns eines kompletten Produkts. Hier durch erreicht man, dass weder Förderorgane unbeabsichtigt beleimt oder besprüht werden, was zu einer nachteiligen Verschmutzung der Maschine führen würde, noch dass es zum Ablösen einzelner Materialschichten in Folge fehlender Beleimung kommt, was zu Maschinenbeschädigungen führen kann.

Eine weitere selbstständige Besonderheit ist ein gezielter Auswurf bzw. ein gezieltes Aussondern einzelner fehlerhafter Produkte. Sobald ein Produkt von einem Sensor als fehlerhaft erkannt worden ist, wird dieses Produkt von der Sensorposition bis zum Aussonderungsorgan verfolgt und dann vom Aussonderungsorgan einzeln und gezielt ausgesondert. Bei herkömmlichen Maschinen ist ein Aussondern nur einer größeren, ein einzelnes fehlerhaftes Produkt enthaltenden Anzahl von Produkten möglich, da Produkte wegen zu geringer Datenverarbeitungsgeschwindigkeiten nicht einzeln verfolgt werden können.

Die obigen Besonderheiten, nämlich die Antriebssteuerung in Abhängigkeit einer Produktkontrolle, das Unterbrechen einer Beleimung nur für die Dauer des Vorbeiförderns einer Fehlstelle und das gezielte Aussondern von fehlerhaften Produkten sind zeitkritische Prozesse, die in Folge der hohen Verarbeitungsgeschwindigkeiten nur bei einer schnellen Datenverarbeitung realisiert werden können. Die erfindungsgemäße Integration von SPS, Achssteuerung und Visualisierung auf einem gemeinsamen physikalischen Gerät ist die Grundlage für die schnelle Datenverarbeitung und damit für diese Besonderheiten.

Weitere Besonderheiten und Einzelheiten der Erfindung werden nachfolgend anhand eines in der Zeichnung dargestellten Ausführungsbeispiels näher erläutert. Es zeigt:
- Fig. 1: ein Blockdiagramm einiger Komponenten einer Maschinensteuerung;
- Fig. 2: eine schematische Darstellung einer Herstellungsmaschine nebst Förderwegen der Produkte, nämlich Pflastern;
- Fig. 3: ein fertiges Pflaster im Querschnitt; und
- Fig. 4: ein fertiges Pflaster im Längsschnitt entlang der Linie IV-IV aus Fig. 3.

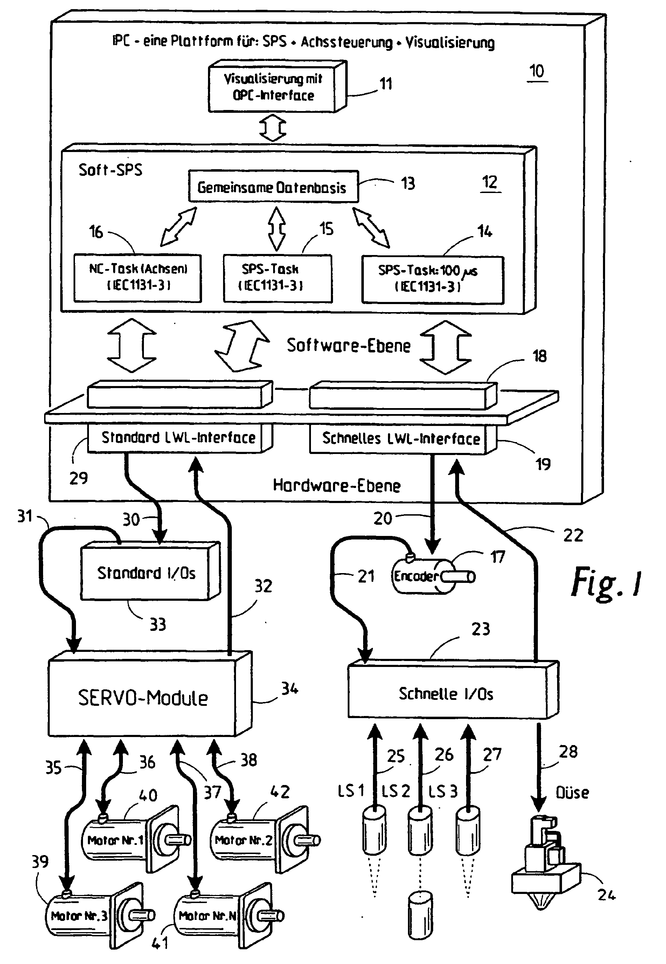

Fig. 1 veranschaulicht die Steuerung einer Produktherstellungsmaschine. Ein als Block dargestelltes gemeinsames physikalisches Gerät 10 bildet eine gemeinsame Plattform für eine SPS, eine Achssteuerung und eine Visualisierung. Bei dem physikalischen Gerät handelt es sich insbesondere um einen Personal Computer IPC nach dem Industriestandard. Ein derartiger Industrie PC hat den Vorteil, kostengünstig zu sein und ständig weiterentwickelt zu werden. Ferner stehen eine Reihe von Entwicklungstools zur Verfügung. Sowohl die Bedienung als auch die ggfs. erforderliche Anpassung dieses Steuerungskonzepts ist somit einfach und kostengünstig zu realisieren.

Ein Visualisierungsmodul 11 dient der Bereitstellung einer Schnittstelle zwischen der Maschine und einem Bediener. Das Visualisierungsmodul 11 stellt u.a. Prozesse und Prozessparameter dar. Es ermöglicht dabei auch die Eingabe von Änderungen der Prozesse bzw. Prozessparameter, bspw. über einen Touch-Screen-Monitor oder ein Bildschirm-Tastatur-System. Das Visualisierungsmodul 11 weist ferner ein sog. OPC (OLE for Process Control)-Interface auf, d.h. eine Schnittstelle zur Prozessteuerung nach dem OLE (Object linking and embedding) - Standard. Das Visualisierungsmodul 11 kommuniziert mit einem sog. Soft-SPS-Modul 12, das eine gemeinsame Datenbasis 13 für eine erste SPS-Task 14 zur Steuerung zeitkritischer Prozesse, eine zweite SPS-Task 15 zur Steuerung nicht-zeitkritischer Prozesse und eine NC-Task 16 zur Steuerung von Achsen, insbesondere von Antrieben, Servomotoren, Förder- und/oder Schneidorganen, bereitstellt.

Die erste SPS-Task 14 erledigt zeitkritische Prozesse, wie die Überwachung einzelner Vorgänge bei der Produktherstellung bzw. -behandlung, bspw. Beleimen und/oder Besprühen einzelner Materialschichten. Ferner erledigt diese SPS-Task 14 auch die Korrektur der Position und Schnittlängen von Material. Die SPS-Task 14 fragt hierzu in einem Takt von 100µs oder schneller eine Position eines Encoders 17 und damit bspw. die Position einer Masterachse sowie die Zustände von bestimmten Ein- und Ausgängen ab und wertet diese in einem Programm aus. Wird eine Positions- bzw. Längenabweichung, bspw. von einem Sollwert bzw. Sollwertbereich, festgestellt, wird von der NC-Task 16 eine Korrektur angefordert.

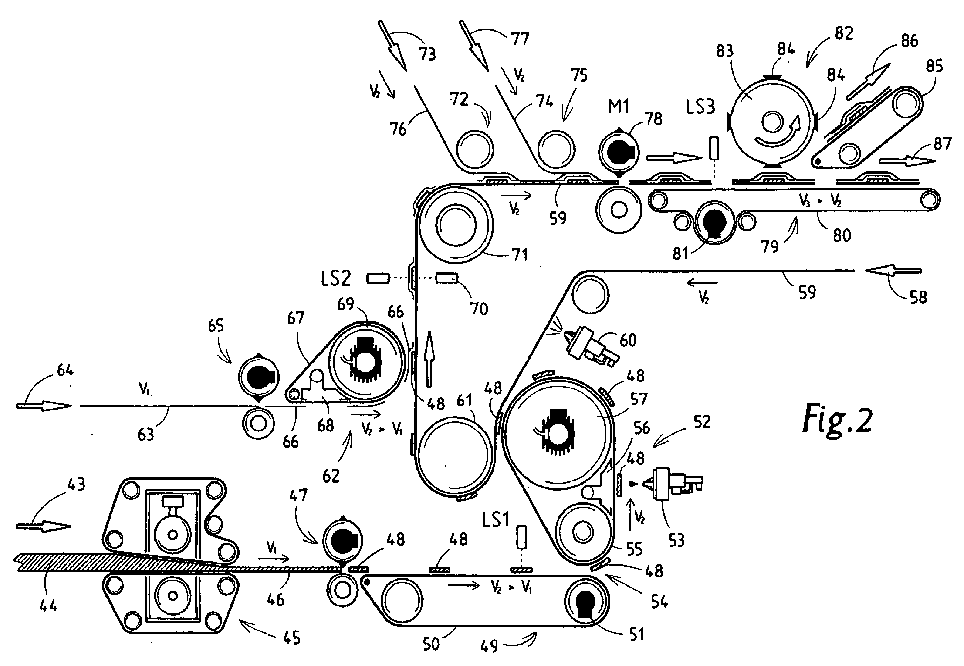

Die NC-Task 16 verwaltet die Ansteuerung von Achsen von Servoantrieben, insbesondere in Form von Start-, Stop- und Positionierungssignalen, sowie eine Kopplung mit der Masterachse und die Fehlerauswertung. Bevorzugt werden einige oder alle rotatorischen Achsen an die Masterachse M1 (siehe Fig. 2) gekoppelt, um die Synchronität der Achsen zueinander zu gewährleisten. Die Positionen bzw. Geschwindigkeiten der Achsen werden automatisch nach Anforderung der ersten SPS-Task 14 korrigiert.

Die zweite SPS-Task 15 erledigt weitere SPS-Aufgaben, die nicht zeitkritisch sind. Hierzu zählen beispielsweise die Steuerung von bestimmten Ventilen, das Abfragen von Türkontaktschaltem, das Ein-/Ausschalten von Lampen und/oder das Überwachen eines Sensors, der einen eventuellen Bandriss anzeigt. Derartige Aufgaben müssen nicht unbedingt in dem schnellen Takt der ersten SPS-Task 14, also alle 100µs ausgeführt werden. Diese Aufgaben können auch in größeren Zeiträumen, beispielsweise nur alle 2-4 ms oder nur jede Sekunde, erledigt werden.

Die Komponenten der Soft-SPS 12, nämlich die gemeinsame Datenbasis 13, erste SPS-Task 14, zweite SPS-Task 15 und NC-Task 16, sind als Software auf dem gemeinsamen physikalischen Gerät 10 bzw. dem IPC realisiert. Insbesondere die erste SPS-Task 14, die zweite SPS-Task 15 und die NC-Task 16 sind in einer einheitlichen Programmiersprache, bspw. IEC1131-3 erstellt, also in einer genormten Programmiersprache (Standard der International Electrotechnical Commission). Das Verwenden derart einheitlicher Programmiersprachen ermöglicht das Eliminieren zusätzlicher Datenprotokollschichten und reduziert somit den Aufwand der Datenverarbeitung. Hierdurch erzielt man eine weitere Geschwindigkeitssteigerung.

Die erste SPS-Task 14 ist auf Software-Ebene mit einem Hardware-Treiber 18 für ein schnelles LWL (Lichtwellenleiter)-Interface 19 verbunden. Das schnelle LWL-Interface 19 schafft eine Verbindung für ein schnelles optisches Bussystem, das mehrere Lichtwellenleiter 20, 21 und 22 aufweist. Ein erster Lichtwellenleiter 20 verbindet das schnelle LWL-Interface 19 mit dem Encoder 17, der die Position der Masterachse M1 ermittelt und auf den Bus gibt. Ein weiterer Lichtwellenleiter 21 verbindet den Encoder 17 mit einer schnelle Ein-/Ausgabe-Baugruppe 23. Diese Baugruppe 23 ist bevorzugt als binäre Baugruppe ausgebildet. Sie weist insbesondere 32 bit Datenbreite auf und kann somit 32 Ein- bzw. Ausgabesignale adressieren. Als Eingabesignale dienen insbesondere die Signale von Sensoren, bspw. Lichtschranken LS1, LS2 und LS3. Bei diesen Eingangssignalen handelt es sich bevorzugt ebenfalls um binäre Signale, die das Vorliegen oder Nichtvorliegen eines bestimmten Zustands anzeigen. Als Ausgabesignal übermittelt die Baugruppe 23 ebenfalls ein binäres Signal, das an ein Behandlungsaggregat, z.B. eine Leimdüse 24 geleitet wird und dieser Leimdüse mitteilt, ob sie geöffnet oder geschlossen sein soll. Die Verbindung zwischen der Baugruppe 23 und den Lichtschranken LS1, LS2 und LS3 sowie der Leimdüse 24 erfolgt insbesondere über elektrische oder optische Leitungen 25 bis 28.

Das Besondere an dem vorstehend beschriebenen optischen Bussystem ist die geringe Anzahl von Teilnehmern an dem Bus. Dieses Bussystem weist nämlich im wesentlichen nur zwei Teilnehmer auf, nämlich den Encoder 17 und die schnelle, binäre Ein-/Ausgabe-Baugruppe 23. Aufgrund dieser geringen Teilnehmeranzahl kann das Bussystem mit nur zwei Telegrammen betrieben werden. Hierdurch vermeidet man zusätzliche che auf den Bus zu übertragende Daten, was die Datenverarbeitungsgeschwindigkeit erhöht.

Das Zusammenspiel des speziellen angepassten Hardware-Treibers 18, des schnellen LWL-Interfaces 19 sowie des optischen Bussystem mit Lichtwellenleitern 20, 21 und 22 mit nur zwei Teilnehmern, nämlich Encoder 17 und Ein-/Ausgabe-Baugruppe 23 erhält man ein sehr schnell arbeitendes Bussystem, das mit einem Takt von 100µs oder schneller abgefragt bzw. betrieben werden kann. Das schnelle LWL-Interface 19 sowie der zugeordnete Hardware-Treiber 18 zeichnen sich dadurch aus, dass sie keinen eigenen Speicher und keinen eigenen Prozessor aufweisen. Vielmehr wird nämlich auf den sehr leistungsstarken Prozessor und den großen Speicher des Industrie PC bzw. des gemeinsamen physikalischen Geräts 10 zugegriffen, was eine Geschwindigkeitserhöhung bewirkt.

Das in Fig. 1 dargestellte Steuerungssystem verfügt jedoch zusätzlich über ein zweites Bussystem. Und zwar kommunizieren die zweite SPS-Task 15 und die NC-Task 16 über ein sog. Standard-LWL-Interface 29 mit diesem zweiten Bussystem. Anders als das schnelle LWL-Interface 19 nebst Hardware-Treiber 18 verfügt das Standard-LWL-Interface über einen eigenen Prozessor nebst entsprechendem Speicher. Dieses Standard-LWL-Interface 29 kann die Kommunikation zwischen der zweiten SPS-Task 15 und der NC-Task 16 und dem zweiten Bussystem selbstständig abwickeln und schont damit Rechnerleistung und Systemresourcen des gemeinsamen physikalischen Geräts 10 bzw. des Industrie PCs. Das Standard-LWL-Interface 29 stellt die Schnittstelle zum zweiten Bussystem dar, das ebenfalls Lichtwellenleiter (LWL) bzw. optische Leitungen 30, 31 und 32 aufweist. Der Lichtwellenleiter 30 verbindet das Standard-LWL-Interface 29 mit einer sog. Standard-Ein-/Ausgabe-Baugruppe 33 zur Übermittlung von digitalen oder analogen Daten an weitere Bussteilnehmer. Insbesondere sieht der Lichtwellenleiter 31 eine Verbindung zwischen der Standard-Ein-/Ausgabe-Baugruppe 33 und mehreren in einem Block 34 zusammengefassten Servo-Modulen mit intelligenten Bussteilnehmern vor. Der Block 34 ist über einen Lichtwellenleiter 32 mit dem Standard-LWL-Interface 29 verbunden. Die einzelnen Servomodule des Blocks 34 stehen wiederum über elektrische Verbindungsleitungen 35, 36, 37 und 38 mit Servomotoren 39, 40, 41 und 42 in Verbindung. Die elektrischen Verbindungsleitungen 35 bis 38 dienen dabei sowohl der Stromversorgung als auch der Übertragung von Steuerungssignalen.

Das aus dem Standard-LWL-Interface 29, der Standard Ein-/Ausgabe-Baugruppe 33 sowie dem Block 34 von Servomodulen und den Lichtwellenleitern 30 bis 32 gebildete zweite Bussystem ist gegenüber dem ersten Bussystem langsamer und dient der Bearbeitung nicht-zeitkritischer Prozesse. Daher genügt ein Aktualisieren der über dieses Bussystem übertragene Daten alle 2 bis 4 ms.

Fig. 2 zeigt eine Maschine zum Herstellen von Pflastern oder anderen einen Zellstoffabschnitt aufweisenden Produkten. Entlang eines ersten Förderwegs 43 wird ein Zellstoffvorprodukt 44 einer Kalandiereinheit 45 zugeführt, die ein Walzwerk mit mehreren Walzen aufweist, zwischen denen das Zellstoffvorprodukt 44 unter starkem Druck hindurchgeführt wird. Erzeugt wird eine Zellstoffbahn 46, die von einem Schneidorgan 47, nämlich einer Messerwalze, in einzelne Zellstoffabschnitte 48 zerschnitten wird. Diese Zellstoffabschnitte 48 dienen im fertigen Produkt zur Aufnahme von Flüssigkeiten, insbesondere von Blut. Sie werden mittels einer Fördereinrichtung 49 mit einem von einer Antriebswalze 51 angetriebenen Förderband 50 in Richtung einer Behandlungseinheit 52 zum Besprühen der Zellstoffabschnitte 48 mit antiseptischem Mittel gefördert. Im Bereich der Fördereinrichtung 49 prüft ein Sensor, nämlich eine Lichtschranke LS1, ob sich ein Zellstoffabschnitt 48 im Bereich der Lichtschranke LS1 befindet. Falls dies der Fall ist, erhält eine Sprühdüse 53 der Behandlungseinheit 52 nach einem definierten Zeitintervall ein Signal, den sich dann im Bereich der Sprühdüse 53 befindenden Zellstoffabschnitt 48 mit antiseptischem Mittel zu besprühen.

In einem Endbereich 54 der Fördereinrichtung 49 werden die Zellstoffabschnitte 48 an die Behandlungseinheit 52 übergeben. Die Behandlungseinheit 52 weist ein sog. Vakuumband 55 und eine Unterdruckeinheit 56 auf. Das Vakuumband 55 ist als luftdurchlässiges Saugband ausgebildet, so dass in Folge des von der Unterdruckeinheit 56 im Inneren des Vakuumbands 55 erzeugten Unterdrucks Zellstoffabschnitte 48 auf dem Band haften bleiben. Auch die Unterdruckeinheit 56 weist mehrere Walzen zum Führen des Vakuumbands 55 auf, von denen wenigstens eine als Antriebswalze 57 ausgebildet ist.

Die Sprühdüse 53 wird über einen Ausgang der schnellen Ein-/Ausgabe-Baugruppe 23 (gemäß Fig. 1) in Abhängigkeit eines von dem Sensor LS1 erzeugten Signals gesteuert. Sie sprüht insbesondere dann kein antiseptisches Mittel in Richtung des Vakuumbandes 55, wenn kein Zellstoffabschnitt 48 vorhanden ist, um das Vakuumband nicht zu beschmutzen. Die Sprühdüse 53 sprüht jedoch auch dann nicht, wenn innerhalb eines Zellstoffabschnitts 48 von dem Sensor LS1 eine Fehlstelle, also beispielsweise ein Loch, erkannt worden ist, um auch dann das Vakuumband 55 nicht zu beschmutzen. Aufgrund der hohen Fördergeschwindigkeiten, insbesondere der Zellstoffabschnitte 48 ist die Sprühdüse derart ausgelegt, dass der Sprühvorgang sehr kurzfristig unterbrechbar ist.

Entlang eines zweiten Förderwegs 58 wird ein Grundkörper 59, bspw. eine Gewebe- oder Textilschicht, gefördert. Dieser Grundkörper 59 bildet bei einem auf der Haut aufgeklebten Pflaster die äußerste Schicht. Dieser Grundkörper wird von einer Sprühdüse 60 mit Sprühleim streifenförmig mit mehreren parallel laufenden Streifen oder flächig, insbesondere ganzflächig, beleimt. Es können jedoch außer einer Sprühdüse auch andere Beleimungsorgane vorgesehen sein. Der beleimte Grundkörper wird derart an einer Walze der Behandlungseinheit 52, insbesondere der Antriebswalze 57 vorbeigefördert, dass ein Zellstoffabschnitt 48 von dem beleimten Grundkörper 59 erfasst und aufgrund der Beleimung mitgenommen wird. Der Abstand zwischen Grundkörper 59 und Vakuumband 55 im Bereich der Antriebswalze 57 ist jedoch vorteilhaft derart gewählt, dass der beleimte Grundkörper nicht mit dem Vakuumband 55 in Kontakt gerät.

Der beleimte Grundkörper mit darauf klebenden Zellstoffabschnitten 48 gelangt über eine Umlenkwalze 61 in den Bereich einer Zuführeinheit 62 zum Zuführen einer weiteren Materiallage, nämlich einer Mullschicht 63. Die Mullschicht 63 wird über einen dritten Förderweg 64 in Richtung der Zuführeinheit 62 transportiert und mittels eines weiteren Schneidorgans 65 in Mullabschnitte 66 geschnitten, die eine derartige Größe aufweisen, dass sie die Zellstoffabschnitte 48 auf dem Grundkörper 59 bedecken. Hierzu werden die Mullabschnitte 66 von der Zuführeinheit 62 ebenfalls mittels eines auf seiner Innenseite eine weitere Unterdruckeinheit 68 aufweisendes und von einer Antriebswalze 69 angetriebenen Vakuumbands 67 mit dem beleimten Grundkörper 59 derart vereinigt, dass zwischen Mullabschnitt 66 und Grundkörper 59 ein Zellstoffabschnitt 48 zu liegen kommt.

Das Vorhandensein eines Zellstoffabschnitts 48 und eines Mullabschnitts 66 wird von einem weiteren Sensor LS2 geprüft. Bei diesem Sensor LS2 handelt es sich bspw. um eine Lichtschranke oder einen mit Laserlicht arbeitenden Lichtvorhang. Die von dem Sensor ausgesandte Lichtintensität ist ausreichend, um Grundkörper 59, Zellstoffabschnitt 48 und Mullabschnitt 66 zu durchleuchten und die jeweilige Lichtintensität auf einem Empfänger 70 des Sensors LS2 empfangen zu können.

Der Sensor LS2 ist ebenfalls ein schnellarbeitender Sensor, der über die schnelle Ein-/Ausgabe-Baugruppe 23 des ersten Bussystems mit der Steuerung verbunden ist. Er arbeitet so schnell, dass eine Positionserfassung der einzelnen Schichten des herzustellenden Pflasters auch bei einer Fördergeschwindigkeit von fünf Metern pro Sekunde mit einer Genauigkeit von 0,5 mm erfassbar ist. Hierdurch können bereits geringfügige Abweichungen der Relativpositionen der einzelnen Schichten des Produkts erkannt und mittels der Steuerung die Geschwindigkeit der einzelnen Antriebsachsen derart geregelt werden, dass eventuelle Abweichungen bei nachfolgenden Produkten wieder reduziert bzw. ausgeglichen werden.

Der nun mit Zellstoffabschnitten 48 und darüber angebrachten Mullabschnitten 63 versehene Grundkörper 59 gelangt nach Durchlaufen einer weiteren Umlenkwalze 71 zunächst zu einer ersten Schutzfolienaufbringungseinheit 72 mittels derer über einen vierten Förderweg 73 eine erste Schutzfolie 74 aufgebracht wird, die etwas mehr als eine Hälfte des Grundkörpers 59 bedeckt. Eine zweite Schutzfolienaufbringungseinheit 75 bringt auf die verbleibende Hälfte des Grundkörpers eine zweite Schutzfolie 76 auf, die entlang eines fünften Förderwegs 77 in Richtung Grundkörper 59 gefördert wird. An dieser Stelle des Verarbeitungsprozesses besteht das Produkt nun aus einem Grundkörper 59 mit darauf angebrachtem Zellstoffabschnitt 48 sowie einem den Zellstoffabschnitt 48 überlappend abdeckenden Mullabschnitt 66, der wiederum durch zwei einander überlappende Schutzfolien bedeckt ist. Sämtliche Schichten werden durch den von der Sprühdüse 60 aufgebrachten Leim am Grundkörper 59 angeklebt.

In Förderrichtung gesehen anschließend, zertrennt ein weiteres Schneidorgan 78 den Grundkörper und zwar im wesentlichen mittig zwischen zwei Zellstoffabschnitten 48. In diesem Bereich entstehen die dann gebrauchsfertigen Pflasterabschnitte, die mittels eines weiteren Sensors LS3 einer Endkontrolle unterzogen werden. Dieser Sensor LS3 ist im Bereich einer Fördereinrichtung 79 mit einem weiteren Förderband 80 und einer Antriebswalze 81 sowie weiteren Umlenkwalzen ausgestattet. Diese Fördereinrichtung dient dem Transport der nunmehr vereinzelten Produkte, nämlich Pflastern.

Falls der Sensor, der wiederum als Lichtschranke oder Lichtvorhang, insbesondere mit Laserlicht arbeitender Lichtvorhang ausgebildet sein kann, ein nicht ordnungsgemäß ausgebildetes Produkt erkennt, aktiviert die Steuerung eine Aussonderungseinrichtung 82, die ein fehlerhaftes Produkt mittels an einer Walze 83 angebrachter Saugnäpfe 84 greift und auf ein Aussonderungsband 85 ablegt. Die derart ausgesonderten Produkte gelangen schließlich über einen sechsten Förderweg 86 in einen nicht dargestellten Ausschussbehälter. Ordnungsgemäß ausgebildete Produkte gelangen über einen siebten Förderweg 87 zum sich anschließenden Verpackungsprozess.

Die Fördergeschwindigkeiten der Förderbänder 50, 80, Vakuumbänder 55, 67 sowie die Zuführgeschwindigkeiten der Zellstoffbahn 46, der Mullschicht 63 sowie der Schutzfolien 74, 76 sind unterschiedlich, um die als Vorprodukte zugeführten einzelnen Pflasterbestandteile in richtigen Abständen positionieren zu können: Die Zellstoffbahn 46 wird mit einer ersten Geschwindigkeit v₁ gefördert. Nach der Vereinzelung durch das Schneidorgan 47 werden die Zellstoffabschnitte 48 mit einer gegenüber der ersten Geschwindigkeit v₁ größeren zweiten Geschwindigkeit v₂ transportiert. Die Behandlungseinheit 52 fördert die Zellstoffabschnitte 48 ebenfalls mit der zweiten Geschwindigkeit v₂, mit der sich auch der Grundkörper 49 vorwärtsbewegt. Die aufzubringenden Mullabschnitte 66 gelangen ebenfalls mit der niedrigeren ersten Geschwindigkeit v₁ zum Schneidorgan 65. Sie werden jedoch nach Vereinzelung mit der höheren, zweiten Geschwindigkeit v₂ auf den Grundkörper 59 bzw. die Zellstoffabschnitte 48 aufgebracht. Jedenfalls mit der zweiten, höheren Geschwindigkeit gelangen die Schutzfolien auf die Mullabschnitte bzw. den Grundkörper 59. Nach der Vereinzelung mittels Schneidorgan 78 werden die fertigen Pflaster mit einer dritten gegenüber der zweiten Geschwindigkeit v₂ höheren Geschwindigkeit v₃ entlang der Aussonderungseinheit dem weiteren Verpackungsprozess bzw. zu einem Ausschussbehälter zugeführt.

Die genannten Geschwindigkeiten v₁, v₂, v₃ müssen exakt aufeinander abgestimmt sein, da anderenfalls die einzelnen Schichten auseinanderlaufen. Die Abstimmung der individuellen Geschwindigkeiten und Positionen der einzelnen Antriebe bzw. deren Antriebsachsen werden mit der in Fig. 1 beschriebenen Steuerung aufeinander abgestimmt. Die in Fig. 1 schematisch dargestellten Servomotoren 39-42 dienen als Antriebsorgane für die in Fig. 2 dargestellten Organe, insbesondere Förder- und Schneidorgane, aber auch für die Kalandiereinheit 45 und die Aussonderungseinrichtung 82. Aufgrund der sehr hohen Herstellungsgeschwindigkeiten für derartige Produkte fällt eine große Datenmenge von Mess- und Steuerungsdaten an, die nur mittels der erfindungsgemäßen Vorrichtung bzw. dem erfindungsgemäßen Verfahren schritthaltend verarbeitet werden kann. Hierdurch lässt sich eine wesentliche Steigerung der Produktqualität erreichen.

Eine der Antriebsachsen der Förderorgane oder der Schneidorgane dient als Masterachse M1, also als Referenz für weitere Achsen. In Fig. 2 ist dies die Achse des Schneidorgans 78. Grundsätzlich kann jedoch im wesentlichen jede beliebige Achse als Master achse gewählt werden. Die Masterachse ist mit dem Encoder 17 versehen, der Werte über die aktuelle Position der Achse liefert.

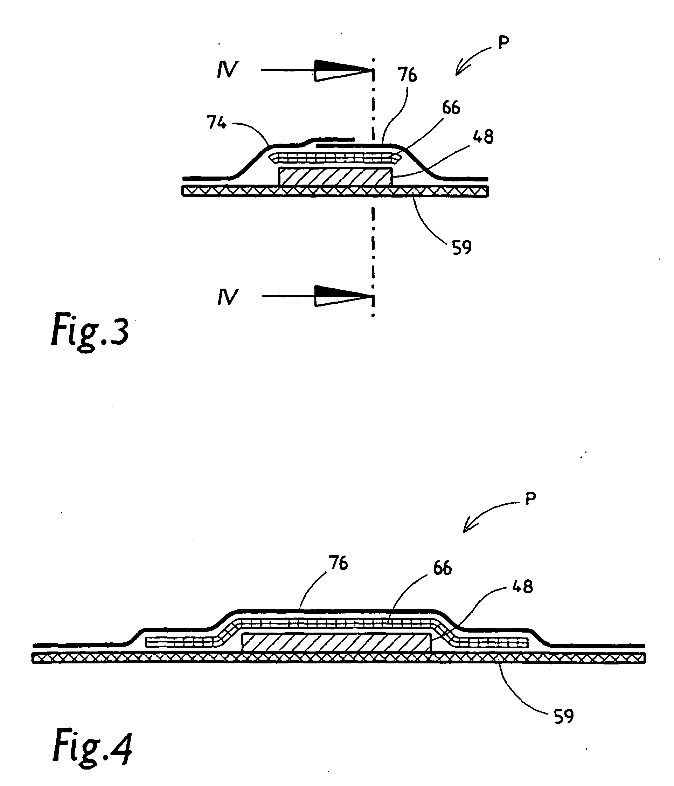

Fig. 3 zeigt ein Pflaster P im Querschnitt, insbesondere quer zur Richtung beispielsweise des siebten Förderwegs. Das fertige Pflaster besteht aus dem Grundkörper 59 mit dem daraufliegenden Zellstoffabschnitt 48. Der Zellstoffabschnitt 48 wird überdeckt mit dem Mullabschnitt 66, der an wenigstens zwei Stellen mit dem Grundkörper 59 verklebt ist. Grundkörper 59, Zellstoffabschnitt 48 und Mullabschnitt 66 werden überdeckt von den Schutzschichten 74, 76, die zum Aufkleben des Pflasters P auf die Haut seitlich abgezogen werden können.

Fig. 4 zeigt das Pflaster P aus Fig. 3 im Längsschnitt, insbesondere in Richtung des siebten Förderwegs gemäß Fig. 2. In Längsrichtung des Pflasters gesehen zeigt Fig. 4, dass der Mullabschnitt 66 den Zellstoffabschnitt 48 vollständig überdeckt. Hierdurch wird eine Fixierung des Mullabschnitts 66 auf dem Grundkörper 59 an den beiden Endbereichen des Mullabschnitts 66 gewährleistet.

Eine nicht dargestellte Besonderheit besteht darin, die Produkte, insbesondere die unfertigen Produkte während des Herstellungsprozesses, vorzugsweise die Zellstoffabschnitte 48, den Grundkörper 59, die Mullabschnitte 66 und/oder die Schutzfolien 74, 76 mit bei Tageslicht sichtbaren oder nicht-sichtbaren Marken zu versehen, um die Positionen der genannten Produktteile mittels der Sensoren besser erfassen zu können. Bspw. könnte eine nur bei UV-Licht sichtbare Farbe aufgetragen werden, die eine Positionserfassung mittels Lichtschranken erleichtert.

Die erläuterten Schneidorgane 47, 65, 78 können mit einem oder mehreren umlaufenden Messern ausgebildet sein. Alternativ oder zusätzlich besteht jedoch auch die Möglichkeit, dass diese Schneidorgane mit einem scharf gebündelten Wasserstrahl das jeweilige Material durchtrennen. Dies ist insbesondere beim Schneiden der Zellstoffbahn 46 vorteilhaft, um die sich einstellende Staubentwicklung zu begrenzen.

Weitere Vorteile der Erfindung liegen darin, dass aufgrund der durch die Integration von SPS, Absteuerung und Visualisierung erreichten übersichtlichen Datenstruktur voll automatische Prozesskorrekturen und vollautomatische Formatwechsel möglich sind. Hierdurch kann auch der Wartungsaufwand einer derartigen Herstellungsmaschine minimiert werden. Aufgrund der erreichbaren offenen Steuerungssystem-Struktur ist auch eine weitgehende Hardwareunabhängigkeit, sowie eine schnelle Anpassbarkeit und eine Steigerung der Systemleistung zusammen mit der Industrie-PC-Entwicklung möglich.

### Bezugszeichenliste:

- 10: gemeinsames physikalisches Gerät
- 11: Visualisierungsmodul
- 12: Soft-SPS-Modul
- 13: Datenbasis
- 14: erste SPS-Task
- 15: zweite SPS-Task
- 16: NC-Task
- 17: Encoder
- 18: Hardware Treiber
- 19: schnelles LWL-Interface
- 20: Lichtwellenleiter
- 21: Lichtwellenleiter
- 22: Lichtwellenleiter
- 23: schnelle Ein-/Ausgabe-Baugruppe
- 24: Leimdüse
- 25: elektrische Leitung
- 26: elektrische Leitung
- 27: elektrische Leitung
- 28: elektrische Leitung
- 29: Standard LWL-Interface
- 30: Lichtwellenleiter
- 31: Lichtwellenleiter
- 32: Lichtwellenleiter
- 33: Standard Ein-/Ausgabe-Baugruppe
- 34: Block von Servo-Modulen
- 35: elektrische Verbindungsleitung
- 36: elektrische Verbindungsleitung
- 37: elektrische Verbindungsleitung
- 38: elektrische Verbindungsleitung
- 39: Servomotor
- 40: Servomotor
- 41: Servomotor
- 42: Servomotor
- 43: erster Förderweg
- 44: Zellstoffvorprodukt
- 45: Kalandiereinheit
- 46: Zellstoffbahn
- 47: Schneidorgan
- 48: Zellstoffabschnitt
- 49: Fördereinrichtung
- 50: Förderband
- 51: Antriebswalze
- 52: Behandlungseinheit
- 53: Sprühdüse
- 54: Endbereich
- 55: Vakuumband
- 56: Unterdruckeinheit
- 57: Antriebswalze
- 58: zweiter Förderweg
- 59: Grundkörper
- 60: Sprühdüse
- 61: Umlenkwalze
- 62: Zuführeinheit
- 63: Mullschicht
- 64: dritter Förderweg
- 65: Schneidorgan
- 66: Mullabschnitt
- 67: Vakuumband
- 68: Unterdruckeinheit
- 69: Antriebswalze
- 70: Empfänger
- 71: Umlenkwalze
- 72: Schutzfolienaufbringungseinheit
- 73: vierter Förderweg
- 74: erste Schutzfolie
- 75: Schutzfolienaufbringungseinheit
- 76: zweite Schutzfolie
- 77: fünfter Förderweg
- 78: Schneidorgan
- 79: Fördereinrichtung
- 80: Förderband
- 81: Antriebswalze
- 82: Aussonderungseinrichtung
- 83: Walze
- 84: Saugnapf
- 85: Aussonderungsband
- 86: sechster Förderweg
- 87: siebter Förderweg
- IPC: Industrie PC
- M1: Masterachse
- LS1: Lichtschranke
- LS2: Lichtschranke
- LS3: Lichtschranke
- P: Pflaster

## Patentansprüche

1. Verfahren zum Steuern einer Vorrichtung zum Herstellen von Produkten (P), insbesondere Zigaretten, Zigarettenpackungen, Pflastern bzw. Pflasterpackungen, mit einer speicherprogrammierbaren Steuerung - SPS -, einer Achssteuerung zum Steuern von Achsen, insbesondere von Antrieben (51, 57, 69, 81), Servomotoren (39-42), Förder (50, 55, 67, 80)- und/oder Schneidorganen (47, 65, 78), und einer Visualisierung zum Darstellen von Prozessen und/oder Prozessparametern, **dadurch gekennzeichnet, dass** die SPS, die Achssteuerung und die Visualisierung auf einem gemeinsamen physikalischen Gerät (10), insbesondere einer gemeinsamen Hardwareplattform, ablaufen, und dass eine erste SPS-Task (14) zeitkritische Prozesse, eine zweite SPS-Task (15) nicht-kritische Prozesse und eine NC-Task (16) die Achsen steuert, wobei diese Tasks auf eine gemeinsame Datenbasis (13) zugreifen.

2. Verfahren nach Anspruch 1, **dadurch gekennzeichnet, dass** die Produkte (P) einzeln von Sensoren (LS1, LS2, LS3, LS4) entlang wenigstens eines Förderwegs (44, 58, 64, 73, 77, 86, 87) der Produkte erfasst und die Achsen permanent in Abhängigkeit von erfassten Produktpositionen geregelt werden.

3. Verfahren nach einem der vorhergehenden Ansprüche, **dadurch gekennzeichnet, dass** die erste SPS-Task (14) zeitkritische Prozesse mit einem Takt von höchstens 100µs ausführt, wobei innerhalb eines Taktes wenigstens eine Position, insbesondere eines Produkts (P) bzw. Produktabschnitts (48, 66), zweier Produktabschnitte zueinander und/oder einer Achse abgefragt, ausgewertet und beim Feststellen einer Abweichung von einem Sollwert bzw. Sollwertebereich von der NC-Task eine Korrektur angefordert wird.

4. Verfahren nach einem der vorhergehenden Ansprüche, **dadurch gekennzeichnet, dass** ein Sensor (LS1, LS2, LS3, LS4) ein Fehlersignal zum Anzeigen eines als vom Sensor fehlerhaft erkannten Produkts (P) erzeugt, wobei dieses Produkt entlang wenigstens eines Förderwegs (48, 58, 64, 73, 77) von der Position des Sensors bis zu einer Aussonderungseinrichtung (82) einzeln verfolgt und bei Erreichen der Aussonderungseinrichtung das betreffende Produkt (P) einzeln und gezielt ausgesondert wird.

5. Verfahren nach einem der vorhergehenden Ansprüche, **dadurch gekennzeichnet, dass** ein Behandlungsprozess, insbesondere Besprüh- und/oder Beleimungsprozess, einer Materialbahn (59) und/oder des Produkts (P) bzw. Produktabschnitts (48) unterbrochen wird, falls ein Sensor (LS1, LS2, LS3, LS4) eine planmäßige oder unplanmäßige Fehlstelle der Materialbahn bzw. des Produkts bzw. -abschnitts anzeigt.

6. Vorrichtung zum Herstellen von Produkten (P), insbesondere Zigaretten, Zigarettenpackungen, Pflastern bzw. Pflasterpackungen, mit einer speicherprogrammierbaren Steuerung - SPS -, einer Achssteuerung zum Steuern von Achsen, insbesondere von Antrieben (51, 57, 69, 81), Servomotoren (39-42), Förder- (50, 55, 67, 80)- und/oder Schneidorganen (47, 65, 78), und einer Visualisierung zum Darstellen von Prozessen und/oder Prozessparametern, **dadurch gekennzeichnet, dass** die SPS, die Achssteuerung und die Visualisierung auf einem gemeinsamen physikalischen Gerät (10), insbesondere einer gemeinsamen Hardwareplattform, realisiert sind, und dass das physikalische Gerät (10) einen Datenspeicher als gemeinsame Datenbasis (13) für eine erste SPS-Task (14) zur Steuerung zeitkritischer Prozesse, eine zweite SPS-Task (15) zur Steuerung nicht-zeitkritischer Prozesse und eine NC-Task (16) zur Achssteuerung aufweist.

7. Vorrichtung nach Anspruch 6, **gekennzeichnet durch** einen wenigstens einen Sensor (LS1, LS2, LS3, LS4) zur Produktpositionserfassung aufweisenden Regelkreis, wobei die Achsen von Förderorganen (50, 55, 67, 80) zum Produktfördem bzw. zum Materialzuführen entsprechend der von dem Sensor (LS1, LS2, LS3, LS4) erfassten Position entlang wenigstens eines Förderwegs (44, 58, 64, 73, 77, 86, 87), insbesondere hinsichtlich Start und Stop eines Drehens, ihrer Umdrehungsgeschwindigkeit und/oder Phasenlage, geregelt werden.

8. Vorrichtung nach Anspruch 6 oder 7, **gekennzeichnet durch** einen Sensor (LS1, LS2, LS3, LS4) zum Erfassen der Positionen von mehreren übereinanderliegenden Materiallagen, insbesondere Karton und aufzubringenden Druckträgern bzw. Grundkörper (59) und einem aufzubringenden Abschnitt (48, 66), insbesondere Zellstoff- bzw. Mullabschnitt, insbesondere Relativposition der Materiallagen, insbesondere als Laserlichtreflexielste bzw. Lichtvorhang ausgebildete Sensoren.

9. Vorrichtung nach einem der Ansprüche 6 bis 8, **gekennzeichnet durch** wenigstens ein Behandlungsaggregat (52, 53, 60), insbesondere Sprüh- oder Leimaggregat, mit einer steuerbaren Düse und einem dem Aggregat zugeordneten Sensor (LS1) zum Erkennen von planmäßigen und unplanmäßigen Fehlstellen an einer Materialbahn (59) bzw. eines Produkts (P) bzw. Produktabschnitts (48), um bei Vorliegen einer Fehlstelle ein Besprühen bzw. Leimaufbringen zu unterbrechen.

10. Vorrichtung nach einem der Ansprüche 6 bis 9, **gekennzeichnet durch** ein erstes Lichtwellenleiter-Interface (19) zur Kommunikation der ersten SPS-Task (14) mit einem ersten optischen Bussystem (20-23) mit einem an einer Achse, insbesondere Masterachse (M1), gekoppelten Encoder (17) zur Positionserfassung der Achse und einer, insbesondere binären, Ein-/Ausgabebaugruppe (23) zum Bereitstellen von, insbesondere binären, Anschlüssen für Sensoren (LS1, LS2, LS3, LS4), insbesondere Lichtschranken und/oder Aggregate (24, 53, 60), insbesondere Sprüh- und/oder Leimaggregate.

11. Vorrichtung nach einem der Ansprüche 6 bis 10, **gekennzeichnet durch** ein zweites Lichtwellenleiter-Interface (29) zur Kommunikation der zweiten SPS-Task (15) mit einem zweiten optischen Bussystem (30-34) mit einer zweiten Ein-/Ausgabebaugruppe (33) und weiteren Modulen (34) zur Steuerung von Servomotoren (39-42).

12. Vorrichtung nach einem der Ansprüche 6 bis 11, **gekennzeichnet durch** wenigstens einen Sensor (LS1, LS2, LS3, LS4), insbesondere eine Lichtschranke, zum Erfassen und/oder Kontrollieren der Produkte (P) an wenigstens einer Position entlang eines Förderwegs der Produkte (P), wobei der Sensor mit der ersten Ein-/Ausgabebaugruppe (23) verbunden ist, und einer Aussonderungseinrichtung mittels derer ein einzelnes, als fehlerhaft erkanntes Produkt (P) gezielt aussonderbar ist.

13. Vorrichtung nach einem der Ansprüche 6 bis 12, **gekennzeichnet durch** Mittel zum Erzeugen von bei Tageslicht sichtbaren oder nicht-sichtbaren Marken auf definierten Bereichen der Produkte, wobei ein oder mehrere Sensoren auf die Marken empfindlich reagieren.

## Claims

1. A method of controlling an apparatus for producing products (P), in particular cigarettes, cigarette packs, plasters or plaster packs, having a programmable logic controller - PLC -, an axis control system for controlling axes, in particular of drives (51, 57, 69, 81), servo motors (39-42), conveyors (50, 55, 67, 80) and/or cutting elements (47, 65, 78), and a visualization system for displaying processes and/or process parameters, **characterized in that** the programmable logic controller, the axis control system and the visualization system run on a common physical appliance (10), in particular a common hardware platform, and that a first PLC task (14) controls time-critical processes, a second PLC task (15) controls non-critical processes and an NC task (16) controls the axes, these tasks making access to a common database (13).

2. The method according to Claim 1, **characterized in that** the products (P) are registered individually by sensors (LS1, LS2, LS3, LS4) along at least one conveying path (44, 58, 64, 73, 77, 86, 87) of the products, and that the axes are continuously regulated on the basis of registered product positions.

3. The method according to one of the preceding Claims, **characterized in that** the first PLC task (14) executes time-critical processes at a clock rate of at most 100µs, it being the case that within one clock period, at least one position, in particular of a product (P) or product section (48, 66), of two product sections in relation to each other and/or of an axis are interrogated, evaluated and, if a deviation from a set point or set-point range is established, a correction is requested of the NC task.

4. The method according to one of the preceding Claims, **characterized in that** a sensor (LS1, LS2, LS3, LS4) generates an error signal to indicate a product (P) detected as faulty by the sensor, this product being tracked individually along at least one conveying path (48, 58, 64, 73, 77) from the position of the sensor as far as a separating device (82) and, when the separating device is reached, the relevant product (P) is separated out individually and deliberately.

5. The method according to one of the preceding Claims, **characterized in that** a treatment process, in particular a spraying and/or gluing process, of a material web (59) and/or of the product (P) or product section (48) is interrupted if a sensor (LS1, LS2, LS3, LS4) indicates a planned or unplanned fault in the material web or the product or product section.

6. An apparatus for producing products (P), in particular cigarettes, cigarette packs, plasters or plaster packs, having a programmable logic controller - PLC -, an axis control system for controlling axes, in particular of drives (51, 57, 69, 81), servo motors (39-42), conveyors (50, 55, 67, 80) and/or cutting elements (47, 65, 78), and a visualization system for displaying processes and/or process parameters, **characterized in that** the PLC, the axis control system and the visualization system are implemented on a common physical appliance (10), in particular a common hardware platform, and that the physical appliance (10) has a data store as a common database (13) for a first PLC task (14) for controlling time-critical processes, a second PLC task (15) for controlling non-time-critical processes and an NC task (16) for axis control.

7. The apparatus according to Claim 6, **characterized by** a control loop having at least one sensor (LS1, LS2, LS3, LS4) for registering product positions, with the axes of conveying elements (50, 55, 67, 80) for conveying products or for feeding material being regulated in accordance with the position, registered by the sensor (LS1, LS2, LS3, LS4), along at least one conveying path (44, 58, 64, 73, 77, 86, 87), in particular with regard to the starting and stopping of a rotation, their speed of revolution and/or phase angle.

8. The apparatus according to Claim 6 or 7, **characterized by** a sensor (LS1, LS2, LS3, LS4) for registering the positions of a plurality of material layers lying one above another, in particular board and print carriers or base elements (59) to be applied, and a section (48, 66) to be applied, in particular cellulose or gauze section, in particular a relative position of the material layers, in particular sensors constructed as a laser-light reflection strip or light curtain.

9. The apparatus according to one of the Claims 6 to 8, **characterized by** at least one treatment unit (52, 53, 60), in particular a spraying or gluing unit, having a controllable nozzle and a sensor (LS1) associated with the unit for detecting planned and unplanned faults on a material web (59) or a product (P) or product section (48), in order to interrupt spraying or application of glue when there is a fault present.

10. The apparatus according to one of the Claims 6 to 9, **characterized by** a first optical fibre interface (19) for communication between the first PLC task (14) and a first optical bus system (20-23) with an encoder (17) coupled to an axis, in particular a master axis (M1), for registering the position of the axis and an input/output subassembly (23), in particular a binary subassembly, for providing connections, in particular binary connections, for sensors (LS1, LS2, LS3, LS4), in particular light barriers and/or units (24, 53, 60), in particular spraying and/or gluing units.

11. The apparatus according to one of the Claims 6 to 10, **characterized by** a second optical fibre interface (29) for communication between the second PLC task (15) and a second optical bus system (30-34) with a second input/output subassembly (33) and further modules (34) for controlling servo motors (39-42).

12. The apparatus according to one of the Claims 6 to 11, **characterized by** at least one sensor (LS1, LS2, LS3, LS4), in particular a light barrier, for registering and/or monitoring the products (P) at least one position along a conveying path of the products (P), the sensor being connected to the first input/output subassembly (23), and to a separating device by means of which an individual product (P) detected as faulty can deliberately be separated out.

13. The apparatus according to one of the Claims 6 to 12, **characterized by** means for producing marks, visible or invisible under daylight, on defined areas of the products, with one or more sensors reacting sensitively to the marks.

## Revendications

1. Procédé de commande d'un dispositif pour la fabrication de produits (P), notamment des cigarettes, des paquets de cigarettes, des pansements ou des boîtes de pansements, à l'aide d'un automate programmable industriel (API), d'une commande d'axe pour commander des axes, notamment d'entraînements (51, 57, 69, 81), de servomoteurs (39 à 42), d'organes de manutention (50, 55, 67, 80) et/ou de découpe (47, 65, 78), et à l'aide d'une visualisation pour la représentation de processus et/ou de paramètres de processus, **caractérisé en ce que** l'API, la commande d'axe et la visualisation se déroulent sur un appareil physique commun (10), notamment une plate-forme matérielle commune, et **en ce qu'**une première tâche d'automate programmable (14) commande des processus prioritaires, une seconde tâche d'automate programmable (15) commande des processus non prioritaires et une tâche de commande numérique (16) commande les axes, ces tâches ayant accès à une base de données commune (13).

2. Procédé selon la revendication 1, **caractérisé en ce que** les produits (P) sont détectés individuellement par des capteurs (LS1, LS2, LS3, LS4) le long d'au moins un parcours de transport (43, 58, 64, 73, 77, 86, 87) des produits et **en ce que** les axes sont réglés de manière permanente en fonction de positions détectées des produits.

3. Procédé selon l'une quelconque des revendications précédentes, **caractérisé en ce que** la première tâche d'automate programmable (14) exécute des processus prioritaires avec un cycle d'au maximum 100 µs, dans un cycle, au moins une position, notamment d'un produit (P), ou d'une partie d'un produit (48, 66), de deux parties d'un produit l'une par rapport à l'autre, et/ou d'un axe, étant consultée, exploitée et une correction étant demandée par la tâche de commande numérique, lors de la constatation d'un écart par rapport à une valeur de consigne ou un intervalle de valeurs de consigne.

4. Procédé selon l'une quelconque des revendications précédentes, **caractérisé en ce qu'**un capteur (LS1, LS2, LS3, LS4) génère un signal d'erreur pour indiquer un produit (P) reconnu comme défectueux par le capteur, ce produit étant suivi individuellement le long d'au moins un parcours de transport (43, 58, 64, 73, 77) depuis la position du capteur jusqu'à une installation de tri (82) et le produit (P) concerné étant trié de manière individuelle et ciblée lors de son arrivée à l'installation de tri.

5. Procédé selon l'une quelconque des revendications précédentes, **caractérisé en ce qu'**un processus de traitement, notamment un processus de pulvérisation et/ou d'encollage, d'une bande de matériau (59) et/ou du produit (P) ou de la partie du produit (48) est interrompu dans le cas où un capteur (LS1, LS2, LS3, LS4) indique une absence systématique ou non systématique de la bande de matériau ou du produit ou de la partie du produit.

6. Dispositif pour la fabrication de produits (P), notamment des cigarettes, des paquets de cigarettes, des pansements ou des boîtes de pansements, à l'aide d'un automate programmable industriel (API), d'une commande d'axe pour commander des axes, notamment d'entraînements (51, 57, 69, 81), de servomoteurs (39 à 42), d'organes de manutention (50, 55, 67, 80) et/ou de découpe (47, 65, 78), et à l'aide d'une visualisation pour la représentation de processus et/ou de paramètres de processus, **caractérisé en ce que** l'API, la commande d'axe et la visualisation sont exécutés sur un appareil physique commun (10); notamment une plate-forme matérielle commune, et **en ce que** l'appareil physique (10) est pourvu d'une mémoire servant de base de données commune (13) pour qu'une première tâche d'automate programmable (14) commande des processus prioritaires, qu'une seconde tâche d'automate programmable (15) commande des processus non prioritaires et qu'une tâche de commande numérique (16) commande les axes.

7. Dispositif selon la revendication 6, **caractérisé par** une boucle d'asservissement pourvue d'au moins un capteur (LS1, LS2, LS3, LS4) pour la détection de la position d'un produit, les axes d'organes de manutention (50, 55, 67, 80) étant réglés pour la manutention du produit ou pour le guidage du matériau, conformément à la position détectée par le capteur (LS1, LS2, LS3, LS4) le long d'au moins un parcours de transport (43, 58, 64, 73, 77, 86, 87), notamment en ce qui concerne le démarrage et l'arrêt d'une rotation, leur vitesse de rotation et/ou une relation de phase.

8. Dispositif selon la revendication 6 ou 7, **caractérisé par** un capteur (LS1, LS2, LS3, LS4), notamment des capteurs représentés comme une bande de réflexion de faisceau laser ou comme un rideau de lumière, pour détecter les positions de plusieurs couches de matériau se chevauchant, notamment un carton et des supports d'impression à appliquer ou un corps de base (59) et une partie à appliquer (48, 66), notamment une partie de cellulose ou de gaze, en particulier la position relative des couches de matériau.

9. Dispositif selon l'une quelconque des revendications 6 à 8, **caractérisé par** au moins un agrégat de traitement (52, 53, 60), notamment un agrégat de pulvérisation ou d'encollage, avec un jet contrôlable et un capteur attribué à l'agrégat (LS1) pour reconnaître une absence systématique ou non systématique d'une bande de matériau (59) ou d'un produit (P) ou d'une partie d'un produit (48) afin d'interrompre une pulvérisation ou un encollage dans le cas d'une absence.

10. Dispositif selon l'une quelconque des revendications 6 à 9, **caractérisé par** une première interface de fibre optique (19) pour la communication de la première tâche d'automate programmable (14), au moyen d'un premier système de bus optique (20 à 23), avec un encodeur (17) couplé à un axe, notamment un axe maître (M1) pour détecter la position de l'axe et avec un module d'entrée/sortie (23), en particulier binaire, pour la mise à disposition de connexions, en particulier binaires, pour les capteurs (LS1, LS2, LS3, LS4), notamment des cellules de détection et/ou des agrégats (24, 53, 60), en particulier des agrégats de pulvérisation et/ou d'encollage.

11. Dispositif selon l'une quelconque des revendications 6 à 10, **caractérisé par** une seconde interface de fibre optique (29) pour la communication de la seconde tâche d'automate programmable (15), au moyen d'un second système de bus optique (30 à 34), avec un second module d'entrée/sortie (33) et des modules additionnels (34) pour la commande de servomoteurs (39 à 42).

12. Dispositif selon l'une quelconque des revendications 6 à 11, **caractérisé par** au moins un capteur (LS1, LS2, LS3, LS4), notamment une cellule de détection, pour détecter et/ou contrôler les produits (P) au niveau d'au moins une position le long d'un parcours de transport des produits (P), le capteur étant connecté au premier module d'entrée/sortie, et d'une installation de tri au moyen de laquelle un produit (P) individuel, reconnu comme étant défectueux, peut être trié de manière ciblée.

13. Dispositif selon l'une quelconque des revendications 6 à 12, **caractérisé par** un moyen pour générer des marques visibles ou non visibles à la lumière du jour sur des zones définies des produits, un ou plusieurs capteurs réagissant de manière sensible aux marques.
